(19) **Europäisches Patentamt / European Patent Office / Office européen des brevets**

(11) **EP 2 238 996 B1**

(12) **EUROPÄISCHE PATENTSCHRIFT**

(45) Veröffentlichungstag und Bekanntmachung des Hinweises auf die Patenterteilung:
**20.02.2019 Patentblatt 2019/08**

(51) Int Cl.:
*A61M 1/34* (2006.01)          *A61M 1/36* (2006.01)
*A61M 1/16* (2006.01)

(21) Anmeldenummer: **10007776.7**

(22) Anmeldetag: **28.04.2005**

(54) **Verfahren zur Bestimmung der Fistelrezirkulation und extrakorporalen Blutbehandlungsvorrichtung**

Method and device for supervising the supply of substitution fluid during an extracorporeal blood treatment

Méthode et dispositif pour surveiller l'adduction de liquide de substitution pendant un traitement extracorporel du sang

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **11.05.2004 DE 102004023080**

(43) Veröffentlichungstag der Anmeldung:
**13.10.2010 Patentblatt 2010/41**

(62) Dokumentnummer(n) der früheren Anmeldung(en) nach Art. 76 EPÜ:
**09009766.8 / 2 108 391**
**05009278.2 / 1 595 560**

(73) Patentinhaber: **Fresenius Medical Care Deutschland GmbH**
**61352 Bad Homburg v.d.H. (DE)**

(72) Erfinder: **Kopperschmidt, Pascal, Dr.**
**97456 Dittelbrunn (DE)**

(74) Vertreter: **Oppermann, Frank et al**
**OANDO Oppermann & Oppermann LLP**
**Wilhelminenstrasse 1a**
**65193 Wiesbaden (DE)**

(56) Entgegenhaltungen:
**EP-A- 1 348 458          WO-A- 00/51664**
**DE-A1- 4 024 434          DE-C1- 19 917 197**

Printed by Jouve, 75001 PARIS (FR)

**Beschreibung**

Die Erfindung bezieht sich auf ein Verfahren zur Bestimmung der Fistelrezirkulation für eine Vorrichtung zur extrakorporalen Blutbehandlung mit einem extrakorporalen Blutkreislauf, der eine erste Kammer eines durch eine Membran in die erste Kammer und eine zweite Kammer unterteilten Dialysators oder Filters einschließt, und einem Flüssigkeitssystem, das die zweite Kammer des Dialysators oder Filters einschließt. Darüber hinaus betrifft die Erfindung eine derartige Vorrichtung zur extrakorporalen Blutbehandlung mit einer Einrichtung zur Bestimmung der Fistelrezirkulation.

Zur Entfernung von harnpflichtigen Substanzen und zum Flüssigkeitsentzug werden beim chronischen Nierenversagen verschiedene Verfahren zur extrakorporalen Blutbehandlung bzw. -reinigung eingesetzt. Bei der Hämodialyse wird das Blut des Patienten außerhalb des Körpers in einem Dialysator gereinigt. Der Dialysator weist eine Blutkammer und eine Dialysierflüssigkeitskammer auf, die von eine semipermeablen Membran getrennt sind. Während der Behandlung strömt das Blut des Patienten durch die Blutkammer. Um das Blut effektiv von harnpflichtigen Substanzen zu reinigen, wird die Dialysierflüssigkeitskammer kontinuierlich von frischer Dialysierflüssigkeit durchströmt.

Während bei der Hämodialyse (HD) der Transport der kleinen molekularen Substanzen durch die Membran im Wesentlichen durch die Konzentrationsunterschiede (Diffusion) zwischen der Dialysierflüssigkeit und dem Blut bestimmt wird, werden bei der Hämofiltration (HF) im Plasmawasser gelöste Substanzen, insbesondere höhermolekulare Stoffe durch einen hohen Flüssigkeitsstrom (Konvektion) durch die Membran des Dialysators effektiv entfernt. Bei der Hämofiltration fungiert der Dialysator als Filter. Eine Kombination aus beiden Verfahren ist die Hämodiafiltration (HDF).

Bei der Hämo(dia)filtration wird ein Teil des durch die Membran abgezogenen Serums durch eine sterile Substitutionsflüssigkeit ersetzt, die entweder stromauf des Dialysators oder stromab des Dialysators dem extrakorporalen Blutkreislauf zugeführt wird. Die Zufuhr der Substitutionsflüssigkeit stromauf des Dialysators wird auch als Predilution und die Zufuhr stromab des Dialysators als Postdilution bezeichnet.

Es sind Vorrichtungen zur Hämo(dia)filtration bekannt, bei denen die Dialysierflüssigkeit online aus Frischwasser und Konzentrationen und die Substitutionsflüssigkeit online aus der Dialysierflüssigkeit hergestellt werden.

Bei den bekannten Hämo(dia)filtrationsvorrichtungen wird die Substitutionsflüssigkeit dem extrakorporalen Blutkreislauf von dem Flüssigkeitssystem der Maschine über eine Substitutionsflüssigkeitsleitung zugeführt. Bei der Predilution führt die Substitutionsflüssigkeitsleitung zu einer Anschlussstelle an der arteriellen Blutleitung stromauf des Dialysators oder Filters, während bei der Postdilution die Substitutionsflüssigkeitsleitung zu einer Anschlussstelle an der venösen Blutleitung stromab des Dialysators führt. Die Substitutionsflüssigkeitsleitung weist im Allgemeinen einen Konnektor auf, mit dem sie entweder an die venöse oder arterielle Blutleitung angeschlossen werden kann. Zum Unterbrechen der Flüssigkeitszufuhr ist an der Substitutionsflüssigkeitsleitung eine Klemme oder dgl. vorgesehen. Ein derartiges Hämo(dia)filtrationsgerät ist beispielsweise aus der EP-A-0 189 561 bekannt.

Die Überwachung der Blutbehandlung setzt die Kenntnis voraus, ob die Substitutionsflüssigkeit stromauf oder stromab des Dialysators oder Filters dem extrakorporalen Blutkreislauf zugeführt wird. Beispielsweise spielt die Pre- und Postdilution eine Rolle für die auf einer Leitfähigkeitsmessung beruhenden Online-Clearance-Messung (OCM), da die Leitfähigkeit der Dialysierflüssigkeit stromab des Dialysators davon abhängig ist, ob eine Pre- oder Postdilution erfolgt.

Die EP-A-1 348 458 A1 beschreibt ein Verfahren und eine Vorrichtung zur Überwachung der Zufuhr von Substitutionsflüssigkeit für eine extrakorporale Blutbehandlungsvorrichtung. Für die Detektion der Zufuhr von Substitutionsflüssigkeit stromauf oder stromab des Dialysators oder Filters wird die Laufzeit der Druckwellen einer in der Substitutionsflüssigkeitsleitung angeordneten Substituatpumpe gemessen. Die Zufuhr von Substitutionsflüssigkeit stromauf oder stromab des Dialysators oder Filters wird auf der Grundlage der Laufzeitmessung erkannt. Das bekannte Verfahren setzt den Einsatz einer Druckwellen erzeugenden Substituatpumpe voraus.

Die DE 101 15 991 C1 beschreibt eine Vorrichtung zum Erkennen von Stenosen in einem Schlauchleitungssystem. Die Druckschrift schlägt vor, auf eine Stenose (Engstelle) im Schlauchleitungssystem bei einer Änderung des Frequenzspektrums einer sich in dem Schlauchleitungssystem ausbreitenden oszillierenden Drucksignals zu schließen. Das Funktionsprinzip der bekannten Vorrichtung beruht darauf, dass die Ursache für die Änderung des dynamischen Verhaltens des Schlauchleitungssystems die Compliance des Leitungssystems ist, d. h. das elastische Nachgeben unter Druck.

Die DE 199 17 197 C1 offenbart ein Verfahren und eine Vorrichtung zur Ermittlung des Blutflusses in einem Gefäßzugang. Der Erfindung liegt die Aufgabe zu Grunde, ein Verfahren anzugeben, das die Bestimmung der Fistelrezirkulation erlaubt. Darüber hinaus ist eine Aufgabe der Erfindung, eine Vorrichtung zur extrakorporalen Blutbehandlung anzugeben, die über eine Einrichtung verfügt, mit der Fistelrezirkulation bestimmt werden kann.

Die Lösung dieser Aufgaben erfolgt erfindungsgemäß mit den in den Patentansprüchen 1 und 3 angegebenen Merkmalen.

Die Erfindung sieht zwei alternative Ausführungsformen vor, die aber beide auf einer Messung des Drucks im Flüssigkeitssystem stromab des Dialysators oder Filters beruhen. Bei der ersten Ausführungsform wird die Substitutionsflüssigkeit fördernde Substituatpumpe abgeschaltet oder eingeschaltet. Der Erfinder hat erkannt, dass die Veränderung des Drucks nach dem Abschalten und/oder Einschalten der Substituatpumpe einen charakteristischen Verlauf hat. Die Zufuhr von Substitutionsflüssigkeit stromauf oder stromab des Dialysators oder Filters wird auf der Grundlage der Veränderung des Drucks nach dem Abschalten oder Einschalten der Substituatpumpe erkannt. Eine für die Veränderung des Drucks charakteristische Größe ist ein plötzlicher Druckanstieg und/oder Druckabfall.

Grundsätzlich kann die Erkennung nur auf der Veränderung des Drucks nach dem Abschalten oder Einschalten der Substituatpumpe erfolgen. Eine höhere Zuverlässigkeit ist aber dann gegeben, wenn die Erkennung von Pre- oder Postdilution auf der Grundlage der Veränderung des Drucks sowohl beim Abschalten als auch Einschalten der Substituatpumpe oder umgekehrt erfolgt.

Bei einer bevorzugten Ausführungsform, die eine Erkennung von Pre- und Postdilution mit besonders hoher Zuverlässigkeit erlaubt, wird die bereits eingeschaltete Substituatpumpe abgeschaltet und nach Ablauf eines vorgegebenen Zeitintervalls wieder eingeschaltet, wobei der Druck stromab des Dialysators oder Filters gemessen wird. Eine Zufuhr von Substitutionsflüssigkeit stromauf oder stromab des Dialysators oder Filters wird auf der Grundlage des Wechsels zwischen einem vorausgehenden Druckanstieg und einem nachfolgenden Druckabfall oder zwischen einem vorausgehenden Druckabfall und einem nachfolgenden Druckanstieg erkannt.

Wenn eine Abfolge von einem Druckanstieg und einem Druckabfall detektiert wird, wird auf die Zufuhr von Substitutionsflüssigkeit stromab des Dialysators oder Filters geschlossen, während auf die Zufuhr von Substitutionsflüssigkeit stromauf des Dialysators oder Filters geschlossen wird, wenn eine Abfolge von einem Druckabfall und einem Druckanstieg detektiert wird.

Ein Vorteil liegt darin, dass keine Druckpulse ausgewertet werden, die sich im Blutkreislauf fortpflanzen. Daher ist es nicht erforderlich, eine oszillierende Druckpulse erzeugende Substituatpumpe vorzusehen, um zwischen postdilutiver und predilutiver Substitution unterscheiden zu können.

Zur Unterdrückung von Störsignalen wird das Drucksignal vorzugsweise mit einem Tiefpassfilter gefiltert.

Die charakteristische Pulsfolge nach Stop und Start der Substituatpumpe kann auf der Grundlage eines Vergleichs des Drucksignals im Blutkreislauf stromab des Dialysators oder Filters mit einem oberen und unteren Grenzwert erfolgen, der für einen auf die Pre- oder Postdilution zurückzuführenden Druckanstieg oder Druckabfall charakteristisch ist.

Die Einrichtung zur Detektion der Zufuhr von Substitutionsflüssigkeit stromauf oder stromab des Dialysators oder Filters der erfindungsgemäßen extrakorporalen Blutbehandlungsvorrichtung weist eine Steuereinheit zum Abschalten und/oder Einschalten der Substituatpumpe, eine Messeinheit zum Messen des Drucks im Blutkreislauf stromab des Dialysators oder Filters und eine Auswerteinheit auf. Die Auswerteinheit ist derart ausgebildet, dass eine Zufuhr von Substitutionsflüssigkeit stromauf oder stromab des Dialysators oder Filters auf der Grundlage der Veränderung des venösen Drucks nach dem Abschalten und/oder Einschalten der Substituatpumpe erkannt wird.

Die Messeinheit zum Messen des venösen Drucks weist vorzugsweise einen venösen Drucksensor und einen mit dem Signalausgang des Drucksensors verbundenen Tiefpassfilter auf.

Die Auswerteinheit weist vorzugsweise einen Komparator auf, der das Ausgangssignal des Drucksensors zur Detektion eines Druckanstiegs und/oder Druckabfalls mit einem vorgegebenen oberen und/oder unteren Grenzwert vergleicht.

Das erfindungsgemäße Verfahren und die erfindungsgemäße Vorrichtung finden in vorteilhafter Weise bei der Bestimmung der Dialysedosis mittels des sog. Online-Clearance-Monitoring (OCM) und bei eine Substituatvorgabe in Abhängigkeit von dem Blutfluss Verwendung. Das Verfahren und die Vorrichtung liefern darüber hinaus eine Entscheidungshilfe in allen relevanten die Dialyse betreffenden Parametern, wenn eine Unterscheidung zwischen postdilutiver- und predilutiver Substituatgabe notwendig ist. Das Verfahren und die Vorrichtung können auch Anwendung finden bei der Bestimmung des Blutflusses, der Bestimmung der Shunt- oder Fistelrezirkulation, in der Überwachung des relativen Blutvolumens bzw. Hämatokrits, der Ermittlung der Füllvolumina des Dialysators oder Filters sowie der Erkennung des venösen Nadeltyps.

Die meisten für die Detektionseinrichtung erforderlichen Komponenten sind im Allgemeinen in den bekannten Blutbehandlungsvorrichtungen bereits vorhanden. Beispielsweise kann auf den venösen Drucksensor zum Messen des Drucks im Blutkreislauf stromab des Dialysators oder Filters zurückgegriffen werden. Auch steht eine Mikroprozessorsteuerung zur Verfügung. Damit ist der apparative Aufwand relativ gering.

Die zweite Ausführungsform der Erfindung setzt den Einsatz einer Druckwellen erzeugenden Substituatpumpe voraus. Die Zufuhr von Substitutionsflüssigkeit stromauf oder stromab des Dialysators oder Filters wird auf der Grundlage des Vergleichs des sich im Blutkreislauf fortpflanzenden oszillierenden Drucksignals, das auf die Substituatpumpe zurückzuführen ist, mit einem charakteristischen Referenzsignal erkannt. Das erfindungsgemäße Verfahren und die Vorrichtung beruhen darauf, dass das Frequenzspektrum des oszillierenden Drucksignals davon abhängig ist, ob sich das Drucksignal bei Predilution über den Dialysator oder bei Postdilution nicht über den Dialysator fortpflanzt. Auch ändert sich die Amplitude der Druckpulse in Abhängigkeit von Pre- oder Postdilution.

Bei den bekannten Blutbehandlungsvorrichtungen wird das sich im extrakorporalen Blutkreislauf fortpflanzende oszillierende Drucksignal der Substituatpumpe von weiteren Drucksignalen überlagert, die beispielsweise auf die im Blutkreislauf stromauf des Dialysators oder Filters angeordnete Blutpumpe oder im Flüssigkeitssystem angeordnete Einrichtungen zurückzuführen sind, zu denen beispielsweise die Konzentratpumpe, Ultrafiltrationspumpe oder Bilanzkammern zählen. Daher wird aus dem im Blutkreislauf gemessenen Drucksignal das auf die Substituatpumpe zurückzuführende Drucksignal gewonnen. Zur Vermeidung von Messfehlern kann der Dialysator oder Filter auch vom Flüssigkeitssystem abgetrennt werden.

Eine bevorzugte Ausführungsform, die eine Detektion der Zufuhr von Substitutionsflüssigkeit mit besonders hoher Zuverlässigkeit erlaubt, sieht als charakteristisches Referenzsignal das Signal der im extrakorporalen Blutkreislauf stromauf des Dialysators oder Filters angeordneten Blutpumpe vor, die ebenfalls oszillierende Drucksignale erzeugt. Diese Ausführungsform geht von der Annahme aus, dass sich bei Predilution Oszillation der Drucksignale von Blutpumpe und Substituatpumpe, die sich über den Dialysator und das Schlauchsystem fortpflanzen, nur unwesentlich von einander unterscheiden. Bei Postdilution hingegen unterscheiden sich Oszillation der Drucksignale von Substituatpumpe und Blutpumpe deutlich, da sich die Drucksignale der Substituatpumpe nicht über den Dialysator fortpflanzen. Damit ist nicht nur ein relatives, sondern auch absolutes Unterscheidungsmerkmal zwischen Pre- und Postdilution gegeben.

Zur Gewinnung der Drucksignale der Blutpumpe und Substituatpumpe wird vorzugsweise eine Fourier-Analyse des stromab des Dialysators oder Filters gemessenen Drucksignals durchgeführt. Aus den Drucksignalen der Substituatpumpe und Blutpumpe werden vorzugsweise die Amplituden von mindestens zwei Oberschwingungen ermittelt. Aus mindestens einem Quotienten aus mindestens einer Oberschwingung höherer Ordnung und mindestens einer Oberschwingung niedrigerer Ordnung der Substituatpumpe wird mindestens ein erster Bewertungsfaktor und aus mindestens einem Quotienten aus mindestens einer Oberschwingung höherer Ordnung und mindestens einer Oberschwingung niedrigerer Ordnung der Blutpumpe wird mindestens ein zweiter Bewertungsfaktor ermittelt.

In der Praxis hat sich als ausreichend erwiesen, wenn mit der Fourier-Transformation die Koeffizienten der zweiten oder höheren Harmonischen des Drucksignals der Blutpumpe bzw. Substituatpumpe auf den Koeffizienten der ersten Harmonischen des Drucksignals der Blutpumpe bzw. Substituatpumpe normiert werden, um die beiden Bewertungsfaktoren zu bilden.

Auf eine Postdilution wird dann geschlossen, wenn die Differenz zwischen dem ersten Bewertungsfaktor und dem zweiten Bewertungsfaktor größer als ein vorgegebener Grenzwert ist, während auf eine Predilution geschlossen wird, wenn die Differenz zwischen dem ersten Bewertungsfaktor und dem zweiten Bewertungsfaktor kleiner als der vorgegebene Grenzwert ist.

Die Einrichtung zur Detektion der Zufuhr von Substitutionsflüssigkeit der erfindungsgemäßen extrakorporalen Blutbehandlungsvorrichtung weist eine Messeinheit zum Messen des Drucks im Blutkreislauf stromab des Dialysators oder Filters und eine Auswerteinheit auf, die wiederum Mittel zur Gewinnung des auf die Substituatpumpe zurückzuführenden oszillierenden Drucksignals, Mittel zum Vergleich des oszillierenden Drucksignals mit einem charakteristischen Referenzsignal und Mittel zur Detektion von Pre- oder Postdilution auf der Grundlage des Vergleichs des oszillierenden Drucksignals der Substituatpumpe mit dem charakteristischen Referenzsignal aufweist.

Eine Unterscheidung zwischen Pre- oder Postdilution kann zu Beginn der Dialysebehandlung allein mit der Auswertung des Drucksignals der Substituatpumpe, vorzugsweise aber in Verbindung mit dem Drucksignal der Blutpumpe getroffen werden.

Außer der Messeinheit zum Messen des Drucks im extrakorporalen Kreislauf stromab des Dialysators oder Filters, die im allgemeinen Bestandteil der bekannten Blutbehandlungsvorrichtungen ist, werden weitere Hardware-Komponenten nicht benötigt.

Im Folgenden werden die beiden alternativen Ausführungsformen des erfindungsgemäßen Verfahrens sowie der erfindungsgemäßen Blutbehandlungsvorrichtung unter Bezugnahme auf die Figuren näher erläutert.

Es zeigen:

Figur 1　eine erste Ausführungsform einer erfindungsgemäßen Vorrichtung zur extrakorporalen Blutbehandlung mit einer Einrichtung zur Detektion von Pre- und Postdilution in stark vereinfachter schematischer Darstellung,

Figur 2 A　eine prinzipielle Darstellung des zeitlichen Verlaufs des Drucks im extrakorporalen Blutkreislauf stromab des Dialysators oder Filters bei Postdilution,

Figur 2 B　eine prinzipielle Darstellung des zeitlichen Verlaufs des venösen Drucks bei Predilution,

Figur 3 A　den während einer in vitro HDF-Dialysebehandlung gemessenen mittleren venösen Druck als Funktion der Zeit bei Postdilution,

Figur 3 B　den mittleren venösen Druck bei Predilution,

Figur 4　eine zweite Ausführungsform der erfindungsgemäßen extrakorporalen Blutbehandlungsvorrichtung in stark vereinfachter schematischer Darstellung,

Figur 5 A　das Frequenzspektrum der auf die Substituatpumpe und die Blutpumpe zurückzuführenden Druckwellen im extrakorporalen Blutkreislauf und eine Tabelle mit normierten Koeffizienten der Drucksignale der Blutpumpe und der Substituatpumpe bei Postdilution,

Figur 5 B  das Frequenzspektrum der auf die Substituatpumpe und die Blutpumpe zurückzuführ-renden Druckwellen im extrakorporalen Blutkreislauf und eine Tabelle mit normierten Koeffizienten der Drucksignale der Blutpumpe und der Substituatpumpe bei Predilution.

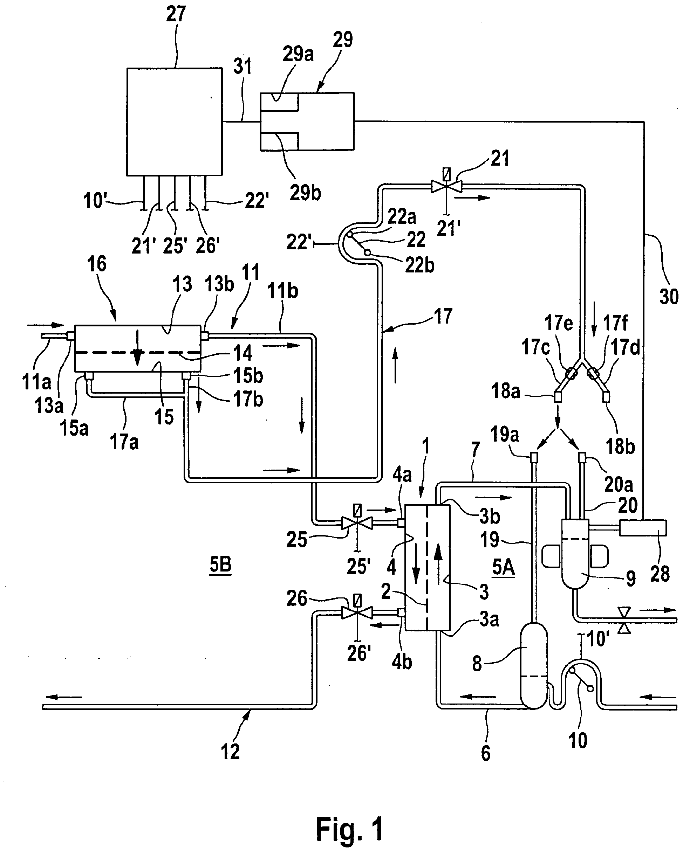

Figur 1 zeigt eine vereinfachte schematische Darstellung der wesentlichen Komponenten einer Hämo(dia)filtrationsvorrichtung zusammen mit einer Einrichtung zur Detektion der Zufuhr von Substitutionsflüssigkeit in den extrakorporalen Blutkreislauf stromauf oder stromab des Dialysators oder Filters. Wenn nachfolgend von einem Dialysator die Rede ist, wird darunter auch ein Filter verstanden.

Die Hämo(dia)filtrationsvorrichtung weist einen Dialysator 1 auf, der durch eine semipermeable Membran 2 in eine von Blut durchflossene erste Kammer 3 und eine von Dialysierflüssigkeit durchflossene zweite Kammer 4 getrennt ist. Die erste Kammer 3 ist in einen extrakorporalen Blutkreislauf 5A geschaltet, während die zweite Kammer 4 in das Flüssigkeitssystem 5B der Hämo(dia)filtrationsvorrichtung geschaltet ist.

Der extrakorporale Blutkreislauf 5A umfasst eine arterielle Blutleitung 6, die zu dem Einlass 3a der Blutkammer 3 führt, und eine venöse Blutleitung 7, die von dem Auslass 3b der Blutkammer 3 des Dialysators 1 abgeht. Zur Eliminierung von Luftblasen sind in die arterielle Blutleitung 6 eine arterielle Tropfkammer 8 und in die venöse Blutleitung 7 eine venöse Tropfkammer 9 geschaltet. Das Blut des Patienten wird durch die Blutkammer des Dialysators mittels einer arteriellen Blutpumpe 10, insbesondere Rollenpumpe gefördert, die an der arteriellen Blutleitung 6 angeordnet ist.

Das Flüssigkeitssystem 5B umfasst eine Dialysierflüssigkeitszuführleitung 11, die zu dem Einlass 4a der Dialysierflüssigkeitskammer 4 führt, und eine Dialysierflüssigkeitsabführleitung 12, die von dem Auslass 4b der Dialysierflüssigkeitskammer 4 des Dialysators 1 abgeht. Über die Dialysierflüssigkeitszuführleitung 11 strömt frische Dialysierflüssigkeit aus einer nicht darge-stellten Dialysierflüssigkeitsquelle in die Dialysierflüssig-keitskammer, während die verbrauchte Dialysierflüssig-keit aus der Dialysierflüssigkeitskammer über die Dialysierflüssigkeitsabführleitung 12 zu einem nicht dargestellten Abfluss abgeführt wird. Die in den Hämo(dia)fil-trationsvorrichtungen im Allgemeinen vorgesehene Bi-lanzierungseinrichtung zur Bilanzierung frischer gegen verbrauchte Dialysierflüssigkeit ist der besseren Übersichtlichkeit halber nicht dargestellt. Auch sind zusätzliche Einrichtungen zum Reinigen und Spülen des Systems nicht dargestellt.

Die Dialysierflüssigkeitszuführleitung 11 umfasst einen ersten Abschnitt 11a, der zu dem Einlass 13a einer ersten Kammer 13 eines durch eine Membran 14 in die erste Kammer und eine zweite Kammer 15 unterteilten Sterilfilters 16 führt, und einen zweiten Abschnitt 11b, der von dem Auslass 13b der ersten Kammer 13 des Filters 16 abgeht und zu dem Einlass 4a der Dialysierflüssigkeitskammer 4 führt.

Während der Dialysebehandlung kann z. B. Dialysierflüssigkeit aus dem Flüssigkeitssystem 5B als Substitutionsflüssigkeit über eine Substitutionsflüssigkeitsleitung 17 dem extrakorporalen Blutkreislauf 5A zugeführt werden. Die Flüssigkeitsleitung 17 weist an beiden Enden jeweils zwei Leitungsabschnitte 17a, 17b, 17c, 17d auf. Der Leitungsabschnitt 17a ist mit dem ersten Auslass 15a des Sterilfilters 16 verbunden, während an den Leitungsabschnitten 17c und 17d jeweils ein Konnektor 18a, 18b angeschlossen ist. Mit den beiden Konnektoren 18a, 18b kann die Substitutionsflüssigkeitsleitung 17 an eine zu der arteriellen Tropfkammer 8 führende Anschlussleitung 19 bzw. eine zu der venösen Tropfkammer 9 führende Anschlussleitung 20 angeschlossen werden. Die Anschlussleitungen 19, 20 verfügen dafür über entsprechende Anschlussstücke 19a, 20a. Auf den Schlauchleitungsabschnitten 17c, 17d sitzen Schlauchklemmen 17e, 17f, mit denen wahlweise eine Flüssigkeitsverbindung nur zu der arteriellen oder venösen Tropfkammer 8, 9 hergestellt werden kann. Es ist aber auch möglich, das die Substitutionsflüssigkeitsleitung 17 mit beiden Anschlussleitungen 19, 20 verbunden und beide Schlauchklemmen 17e, 17f offen sind.

Zum Abklemmen der Substitutionsflüssigkeitsleitung 17 ist stromab des Sterilfilters 16 ein Absperrorgan 21, beispielsweise eine Schlauchklemme vorgesehen, die vorzugsweise elektromagnetisch betätigt wird. Die Substitutionsflüssigkeit wird mittels einer Okklusionspumpe, insbesondere Rollenpumpe 22 gefördert, in die die Substitutionsflüssigkeitsleitung 17 eingelegt ist. Derartige Rollenpumpen gehören zum Stand der Technik. Sie verfügen über mehrere Rollen 22a, 22b, mit denen der Querschnitt der Schlauchleitung zur Förderung der Flüssigkeit verringert werden kann. Dadurch entstehen Druckwellen, die sich in beiden Richtungen über die Substitutionsflüssigkeitsleitung fortpflanzen können. Es sei bemerkt, dass eine Druckwellen erzeugende Okklusionspumpe als Substituatpumpe bei der ersten Ausfüh-rungsform der erfindungsgemäßen Blutbehandlungsvor-richtung nicht erforderlich ist. Vielmehr kann zur Förderung der Substitutionsflüssigkeit auch eine Substituatpumpe Verwendung finden, die Druckwellen nicht erzeugt. Darin liegt ein Vorteil dieser Ausführungsform.

Zur Abkopplung des Dialysators 1 von dem Flüssigkeitssystem 5B sind in der Dialysierflüssigkeitszuführleitung 11 stromauf und in der Dialysierflüssig-keitsabführleitung 12 stromab des Dialysators 1 jeweils ein elektromagnetisch betätigbares Absperrorgan 25, 26 vorgesehen.

Die Blutpumpe 10, die Substituatpumpe 22 sowie die Absperrorgane 21, 25 und 26 sind über Steuerleitungen 10', 22', 21', 25' und 26' mit einer zentralen Steuer- und Regeleinheit 27 verbunden, von der die einzelnen Komponenten unter Berücksichtigung der vorgegebenen Behandlungsparameter angesteuert werden.

Zum Betrieb der Hämo(dia)filtrationsvorrichtung als Hämodialysevorrichtung wird das Absperrorgan 21 geschlossen, wobei Dialysierflüssigkeit durch die Dialysierflüssigkeitskammer 4 des Dialysators strömt. Zum Betrieb der Hämo(dia)filtrationsvorrichtung als Hämodiafiltrationsvorrichtung wird das Absperrorgan 21 geöffnet, so dass aus dem Sterilfilter 16 sterile Dialysierflüssigkeit als Substitutionsflüssigkeit wahlweise in die arterielle Tropfkammer 8 (Predilution) oder venöse Tropfkammer 9 (Postdilution) strömt. Es ist aber auch ein Betrieb der Hämo(dia)filtrationsvorrichtung nur als Hämofiltrationsvorrichtung möglich, wenn der Zufluss von Dialysierflüssigkeit in die Dialysierflüssigkeitskammer 4 des Dialysators unterbrochen wird. Zur Unterbrechung der Flüssigkeitszufuhr wird das Absperrorgan 25 stromauf des Dialysators geschlossen.

Die Einrichtung zur Detektion von Pre- und Postdilution weist eine Steuereinheit auf, die Teil der zentralen Steuer- und Regeleinheit 27 der Blutbehandlungsvorrichtung ist. Darüber hinaus weist die Detektionseinrichtung eine Messeinheit 28 zum Messen des Drucks im extrakorporalen Blutkreislauf stromab des Dialysators 3 und eine Auswerteinheit 29 auf. Als Messeinheit ist ein an der Tropfkammer 9 angebrachter Drucksensor 28 vorgesehen, der ein von dem venösen Druck abhängiges elektrisches Drucksignal erzeugt. Das Drucksignal des Drucksensors 28 wird über eine Datenleitung 30 der Auswerteinheit 29 zugeführt, die wiederum über eine Datenleitung 31 mit der Steuereinheit 27 in Verbindung steht. Die Auswerteinheit 29 weist einen Tiefpassfilter 29a zum Filtern des Drucksignals des Drucksensors 28 und einen Komparator 29b auf, mit dem das Drucksignal mit einem vorgegebenen oberen und unteren Grenzwert verglichen wird.

Nachfolgend wird die Detektion der Zufuhr von Substitutionsflüssigkeit stromauf oder stromab des Dialysators im einzelnen beschrieben. Die Steuereinheit 27 der Detektionseinrichtung schaltet vorzugsweise zu Beginn der Blutbehandlung für die Einleitung der Messung die bereits laufende Substituatpumpe 22 ab. Nach Ablauf eines vorgegebenen Zeitintervalls schaltet die Steuereinheit 27 die Substituatpumpe 22 wieder an. Während die Substituatpumpe ausgeschaltet ist, misst der Drucksensor 28 den venösen Druck. Das mit dem Tiefpassfilter 29a gefilterte venöse Drucksignal des Sensors 28 wird in dem Komparator 29b der Auswerteinheit 29 mit einem oberen und unteren Grenzwert verglichen, um entweder nach dem Abschalten der Substituatpumpe einen Druckanstieg und nach dem Einschalten der Substituatpumpe einen Druckabfall oder nach dem Abschalten der Substituatpumpe einen Druckabfall und nach dem Einschalten der Substituatpumpe einen Druckanstieg zu detektieren.

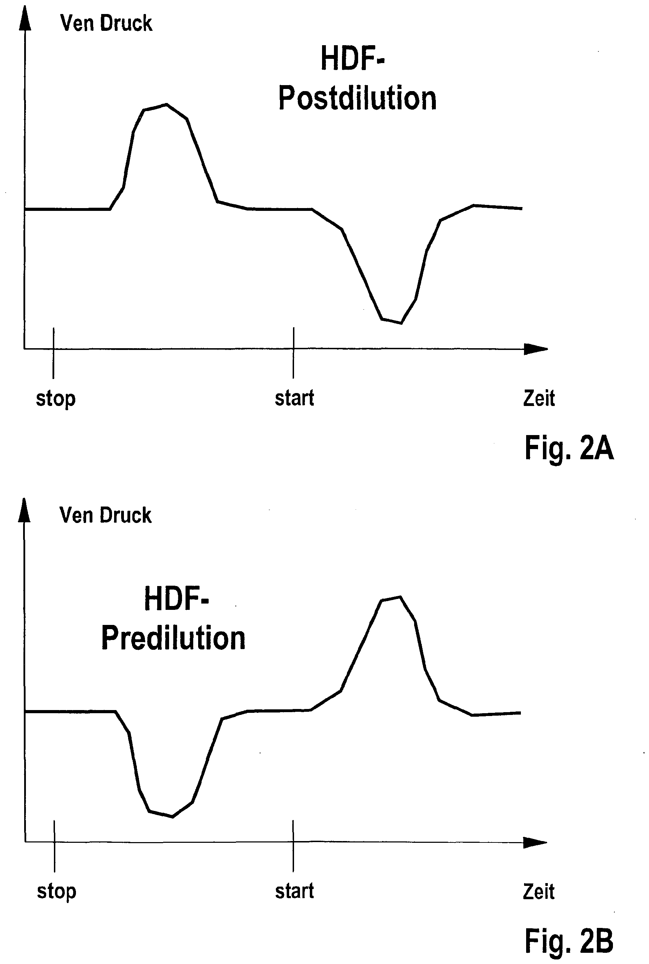

Figur 2A zeigt den prinzipiellen zeitlichen Verlauf des venösen Drucks bei Postdilution und Figur 2B den zeitlichen Verlauf des Drucks bei Predilution. Deutlich ist zu erkennen, dass bei Postdilution der Druck zunächst ansteigt, um anschließend abzufallen. Bei Predilution fällt der Druck zunächst ab, um dann anzusteigen. Auf der Grundlage dieser beiden charakteristischen Verläufe erkennt die Auswerteinheit, ob Postdilution oder Predilution vorliegt. Das Ergebnis kann auf einer nicht dargestellten Anzeigeeinheit optisch und/oder akustisch angezeigt werden.

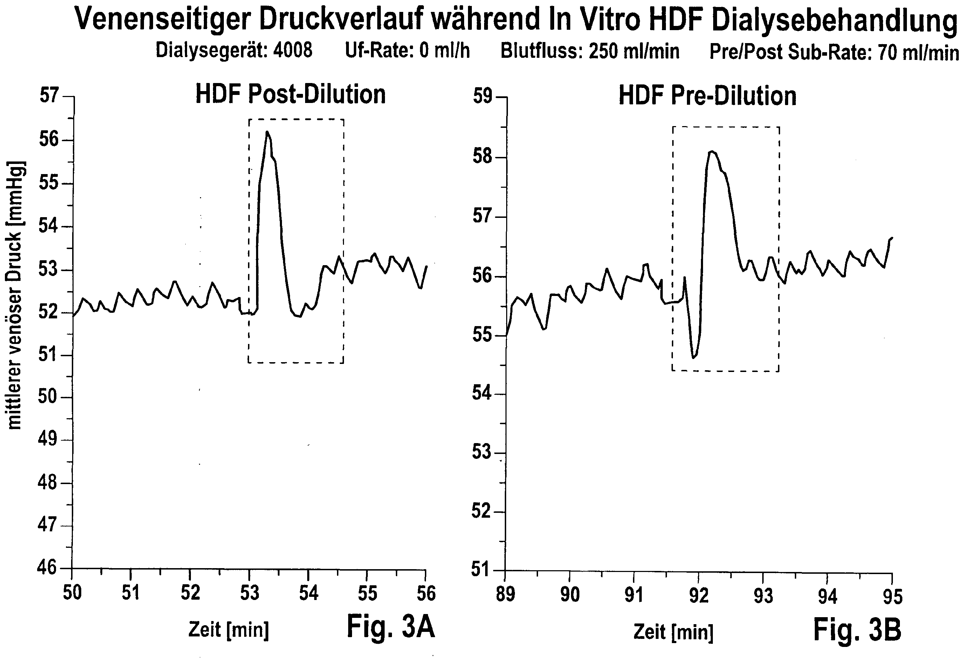

Die Figuren 3A und 3B zeigen den venösen Druck als Funktion der Zeit für den Fall, dass das vorgegebene Zeitintervall, in dem die Substituatpumpe ausgeschaltet ist, relativ lang ist. Wenn ein kürzeres Zeitintervall vorgegebenen wird, liegen die Maxima bzw. Minima näher bei einander. Zur Einleitung der Messung wird die bereits eingeschaltete Substituatpumpe für ein vorgegebenes Zeitintervall ausgeschaltet. Grundsätzlich ist es aber auch möglich, die zunächst ausgeschaltete Substituatpumpe für ein vorgegebenes Zeitintervall einzuschalten.

Der in den Figuren 2A und 2B gezeigte charakteristische Verlauf des Drucksignals ist auf die veränderte Viskosität des Blutes bei Pre- oder Postdilution zurückzuführen. Bei der Postdilution wird die Viskosität des Blutes aufgrund der Zufuhr von Substitutionsflüssigkeit in der venösen Blutleitung 7 stromab der Tropfkammern 9 verringert. Wird die Substituatpumpe 22 angehalten, erhöht sich die Viskosität des Bluts in diesem Abschnitt der venösen Blutleitung. Folglich tritt an der venösen Nadel ein erhöhter Druckabfall auf. Dadurch steigt der venöse Druck an. Wenn die Substituatpumpe wieder eingeschaltet wird, verringert sich die Viskosität des Blutes in der venösen Blutleitung 7 stromab der Tropfkammer 9, so dass der venöse Druck abfällt. Folglich erfolgt nach dem Stop der Substituatpumpe ein Druckpuls mit positivem Vorzeichen und nach dem Start der Pumpe ein Druckpuls mit negativem Vorzeichen. (Fig. 2A). Die Pulsbreite hängt vom Verhältnis des Blutvolumens zwischen der Dialysierflüssigkeitskammer 3 des Dialysators 1 und der Tropfkammer 9 sowie dem Blutfluss ab. Bei Predilution hingegen kehrt sich nach dem Anhalten und Einschalten der Substituatpumpe die Reihenfolge der Vorzeichen des Druckpulses um. Es erfolgt beim Stop der Pumpe ein Druckabfall und nach dem Start der Pumpe ein Druckanstieg (Figur 2B).

Die Figuren 3A und 3B zeigen den mittleren venösen Druck [mmHg] als Funktion der Zeit während einer in vitro HDF-Dialysebehandlung, wobei der Blutfluss auf 250 ml/min, die Substitutionsrate für Pre- und Postdilution auf 70 ml/min und die Ultrafiltrationsrate auf 0 ml/h eingestellt ist. In der Praxis zeigt sich, dass die Amplituden des Druckanstiegs oder Druckabfalls unterschiedlich sind. Der Druckanstieg oder Druckabfall ist aber sowohl bei der Pre- als auch Postdilution deutlich zu erkennen. Die vorgegebenen oberen und unteren Grenzwerte sind derart einzustellen, dass sie unterhalb bzw. oberhalb der Maxima bzw. Minima liegen. Andererseits sollten die oberen und unteren Grenzwerte aber oberhalb bzw. unterhalb der dem Drucksignal überlagerten Druckschwankungen liegen.

Aus den positiven bzw. negativen Druckpulsen kann auch auf die Fistelrezirkulation geschlossen werden. Eine Wiederholung der Druckpulsfolge, die sich in der venösen Blutleitung 7 mit dem venösen Drucksensor 28 nachweisen lässt, in der arteriellen Blutleitung 6, die sich mit einem arteriellen Drucksensor nachweisen lässt, deutet auf eine Rezirkulation hin. Als Maß für die Rezirkulation dient das Verhältnis zwischen dem Integral des Drucksignals des arteriellen Drucksensors in einem vorgegebenen Zeitintervall, in dem der sich wiederholende negative bzw. positive Druckpuls liegt und dem Integral des Drucksignals des venösen Drucksensors 28 in einem vorgegebenen Zeitintervall, in dem der negative bzw. positive Druckpuls liegt. Die Rezirkulation Rez [%] berechnet sich nach der folgenden Gleichung:

$$\mathrm{Re}\, z[\%] = 100 \cdot \left( \frac{\int p_{art} dt}{\int p_{ven} dt} \right)$$

Aus der Zeitdifferenz ($\Delta t$) zwischen dem Beginn und Ende der Druckpulserhöhung oder Druckpulsverringerung kann bei Kenntnis der Blutförderrate ($Q_b$) auf das Füllvolumen ($V_{PD}$) des Schlauchsegments zwischen dem Eingang 3a der Dialysierflüssigkeitskammer 3 des Dialysators 1 und der Anschlussstelle der Substitutionsflüssigkeitsleitung 17, 20 an der Tropfkammer 9 geschlossen werden. Das Volumen berechnet sich nach der folgenden Gleichung:

$$V_{PD} = Q_b \Delta t = Q_b \left( t_{Ende} - t_{Beginn} \right)$$

Darüber hinaus kann auf das Füllvolumen ($V_{PD}$) des Schlauchsegments zwischen dem Eingang 3a der Dialysierflüssigkeitskammer 3 und der venösen Nadel geschlossen werden, wenn bei der HDF-Predilution das vorgegebene Zeitintervall zwischen dem Anhalten der Substituatpumpe 22 und dem Beginn des Druckabfalls bzw. das Zeitintervall zwischen dem Einschalten der Substituatpumpe und dem Beginn des Druckanstiegs mit der Blutförderrate ($Q_b$) multipliziert wird. Bei HDF-Postdilution besteht ein analoger Zusammenhang, wobei die Zeitdifferenz zwischen dem Stop der Substituatpumpe und dem Beginn des Druckpulsanstiegs bzw. die Zeitdifferenz zwischen dem Start der Substituatpumpe und dem Beginn des Druckpulsabfalls überwacht wird.

In umgekehrter Weise kann bei Kenntnis des Füllvolumens jeweils der oben genannten Schlauchsegmente und Zeitdifferenzen der wahre Blutfluss ermittelt werden.

Ist der Zusammenhang zwischen Hämatokrit, Blutdruckabfall bzw. Blutdruckanstieg, Blutförderrate und Nadelgeometrie bekannt, so gibt die Amplitude des Druckanstiegs bzw. -abfalls Aufschluss über den Typ der Nadel. Wenn der Typ der Nadel bekannt ist, kann entsprechend auf den Hämatokrit geschlossen werden.

So kann die Änderung des Hämatokrits während der Dialysebehandlung anhand der Änderung der Amplitude der Druckpulse überwacht werden.

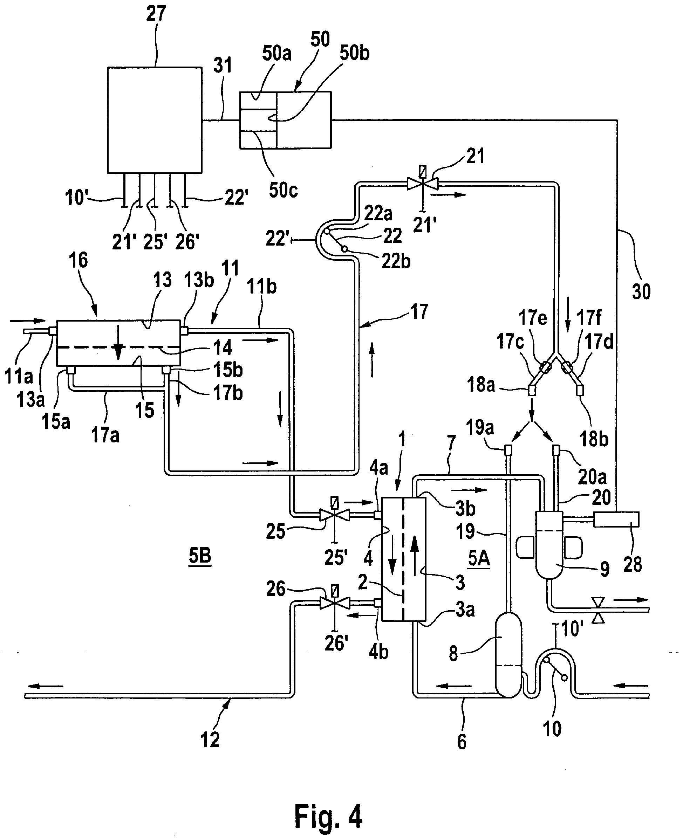

Figur 4 zeigt die alternative Ausführungsform der erfindungsgemäßen Blutbehandlungsvorrichtung in vereinfachter schematischer Darstellung. Mit Ausnahme der Einrichtung zur Detektion von Pre- oder Postdilution unterscheidet sich die Blutbehandlungsvorrichtung von Figur 4 nicht von der Blutbehandlungsvorrichtung von Figur 1. Daher werden die einander entsprechenden Komponenten auch mit den gleichen Bezugszeichen bezeichnet.

Die Detektionseinrichtung weist auch einen venösen Drucksensor 28 zum Messen des Drucks in der venösen Blutleitung 7 auf. Die Auswerteinheit 50 empfängt das venöse Drucksignal des Drucksensors 28 über die Datenleitung 30. Die Auswerteinheit 50 weist eine Einrichtung zur Fourier-Analyse 50a auf, die eine Fourier-Analyse des venösen Drucksignals durchführt.

Das Verfahren zur Detektion von Pre- oder Postdilution setzt voraus, dass die Substituatpumpe 22 und die Blutpumpe 10 Druckwellen erzeugende Pumpen sind, beispielsweise Okklusionspumpen, insbesondere Rollenpumpen. Da die Frequenz der von der Blutpumpe 10 erzeugten Druckwellen infolge der sehr viel höheren Drehzahl der Blutpumpe im Vergleich mit der Substituatpumpe sehr viel größer als die Frequenz der Druckwellen der Substituatpumpe ist, können die Druckwellen der Blutpumpe von denen der Substituatpumpe unterschieden werden. Bei der Predilution durchlaufen die Druckwellen der Substituatpumpe 22 neben dem Schlauchabschnitt der Substitutionsflüssigkeitsleitung 17, 19, 20 den Schlauchabschnitt der arteriellen Blutleitung 6 zwischen Tropfkammer 8 und Eingang 3a der Dialysierflüssigkeitskammer 3, die Dialysierflüssigkeitskammer und die venöse Blutleitung 7, bevor sie den Drucksensor 28 erreichen. Bei der Postdilution durchlaufen die Druckwellen hingegen nicht die Dialysierflüssigkeitskammer und die zugehörigen Blutleitungsabschnitte.

Die Auswirkung der Dialysierflüssigkeitskammer 3 und der dazugehörigen Blutleitungsabschnitte kann durch eine Übertragungsfunktion beschrieben werden. Wenn der Dialysator und die zugehörigen Leitungsabschnitte in dem Übertragungsweg der Druckwellen liegen, ändert sich die Dynamik der Druckpulse und deren Frequenzspektrum, insbesondere werden höhere Frequenzen stärker gedämpft.

Eine genaue Kenntnis der Übertragungsfunktion ist für die Detektion der Pre- oder Postdilution nicht erforderlich. Ausreichend ist, wenn das Drucksignal der Substituatpumpe mit dem Drucksignal der Blutpumpe ins Verhältnis gesetzt wird.

Das mit dem Drucksensor 28 gemessene Drucksignal enthält sowohl das Drucksignal der Blutpumpe 10 als auch das der Substituatpumpe 22. Die Einrichtung zur Fourier-Transformation 50a der Auswerteinheit 50 zerlegt das mit dem Drucksensor 28 gemessene Drucksignal der Blutpumpe 10 und der Substituatpumpe 22 in die auf die Blutpumpe bzw. Substituatpumpe zurückzuführenden Signalkomponenten.

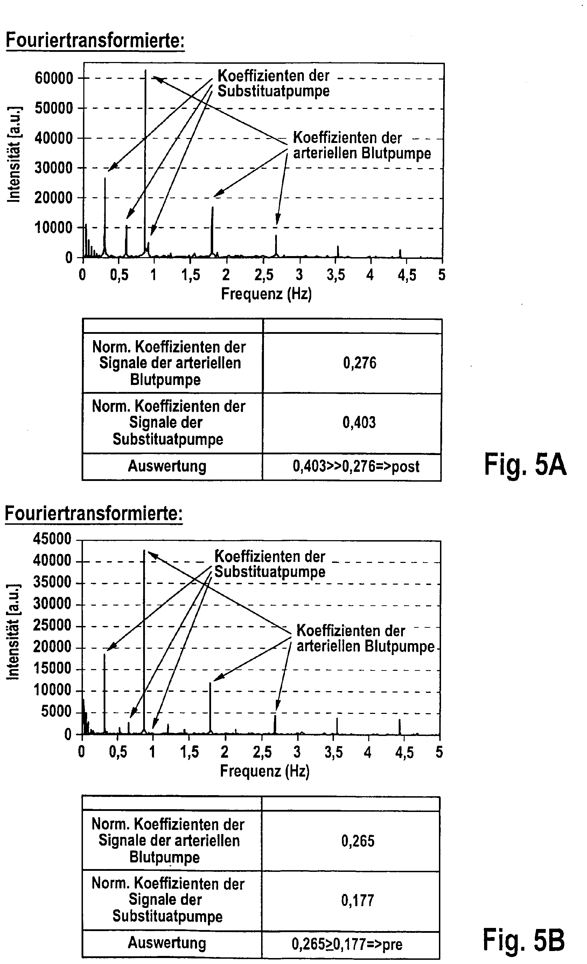

Die Figuren 5A und 5B zeigen das mit der Fourier-Transformation ermittelte Frequenzspektrum des venösen Drucksignals bei Post- bzw. Predilution. Die in den Figuren gezeigten Messwerte wurden bei einem Laborversuch ermittelt, bei dem der Blutfluss auf 300 ml/min, der Substituatfluss auf 80 ml/min und die Ultrafiltrationsrate auf 100 ml/h eingestellt wurde. In dem Frequenzspektrum sind die Koeffizienten der ersten Oberschwingung sowie der Oberschwingungen höherer Ordnung zu erkennen, die auf die Blutpumpe 10 und die Substituatpumpe 22 zurückzuführen sind.

Die Auswerteinheit 50 berechnet den Quotienten zwischen einer Harmonischen höherer Ordnung, beispielsweise der zweiten oder dritten harmonischen und dem Koeffizienten der harmonischen ersten Ordnung, d. h. den normierten Koeffizienten der spektralen Zerlegung. Der normierte Koeffizient der Substituatpumpe stellt einen ersten Bewertungsfaktor dar, während der normierte Koeffizient der Blutpumpe einen zweiten Bewertungsfaktor darstellt.

Zum Vergleichen der Bewertungsfaktoren der Substituatpumpe und der Blutpumpe weist die Auswerteinheit 50 eine weitere Einheit 50b auf. Wenn der Bewertungsfaktor der Substituatpumpe erheblich oberhalb des Bewertungsfaktors der Blutpumpe liegt, wird auf eine Postdilution geschlossen (Fig. 5A). Liegt der Bewertungsfaktor der Substituatpumpe nur oberhalb oder nahe der Substituatpumpe oder sind beide Bewertungsfaktoren sogar gleich, so wird auf eine Predilution geschlossen (Fig. 5B). Die Detektion von Pre- oder Postdilution auf der Grundlage des Vergleichs der Bewertungsfaktoren erfolgt in einer Einrichtung 50c.

Die Einrichtung 50c zum Erkennen von Preoder Postdilution verfügt über eine Einrichtung zur Bildung der Differenz zwischen den beiden Bewertungsfaktoren. Die Differenz wird mit einem vorgegebenen Grenzwert verglichen, der derart festgelegt ist, dass sicher zwischen Pre- oder Postdilution unterschieden werden kann. Eine Postdilution liegt vor, wenn die Differenz zwischen dem ersten und zweiten Bewertungsfaktor größer als der vorgegebene Grenzwert ist, und eine Predilution liegt vor, wenn die Differenz zwischen dem ersten und zweiten Bewertungsfaktor kleiner als der vorgegebene Grenzwert ist. Das Ergebnis kann in einer nicht dargestellten optischen und/oder akustischen Anzeigeeinheit angezeigt werden.

**Patentansprüche**

1. Verfahren zur Bestimmung der Fistelrezirkulation für eine Vorrichtung zur extrakorporalen Blutbehandlung mit einem extrakorporalen Blutkreislauf, der eine erste Kammer eines durch eine Membran in die erste Kammer und eine zweite Kammer unterteilten Dialysators oder Filters einschließt, und einem Flüssigkeitssystem, dass die zweite Kammer des Dialysators oder Filters einschließt, wobei Substitutionsflüssigkeit stromauf oder stromab des Dialysators oder Filters mit einer Substituatpumpe zugeführt wird,

**dadurch gekennzeichnet,**

**dass** die abgeschaltete Substituatpumpe eingeschaltet oder die eingeschaltete Substituatpumpe abgeschaltet wird, und der Druck stromab des Dialysators oder Filters in der venösen Blutleitung des extrakorporalen Blutkreislaufs und der Druck stromauf des Dialysators oder Filters in der arteriellen Blutleitung des extrakorporalen Blutkreislaufs gemessen wird und

**dass** zur Bestimmung der Fistelrezirkulation das Verhältnis zwischen

dem Integral des in der arteriellen Blutleitung gemessenen arteriellen Drucksignals in einem vorgegebenen Zeitintervall nach dem Einschalten bzw. Ausschalten der Substituatpumpe, in dem ein sich wiederholender negativer bzw. positiver arterieller Druckpuls liegt, und

dem Integral des in der venösen Blutleitung gemessenen venösen Drucksignals in einem vorgegebenen Zeitintervall nach dem Einschalten bzw. Ausschalten der Substituatpumpe, in dem ein sich wiederholender negativer bzw. positiver venöser Druckpuls liegt,

berechnet wird.

2. Verfahren nach einem der Anspruch 1, **dadurch gekennzeichnet, dass** der Druck im Blutkreislauf mit einem Drucksensor stromab des Dialysators oder Filters gemessen wird, dessen Ausgangssignal mit einem Tiefpassfilter gefiltert wird.

3. Vorrichtung zur extrakorporalen Blutbehandlung mit einem extrakorporalen Blutkreislauf (5A), der eine erste Kammer (3) eines durch eine Membran (2) in die erste Kammer und eine zweite Kammer (4) unterteilten Dialysators (1) oder Filters einschließt, und einem Flüssigkeitssystem (5B), das die zweite Kammer des Dialysators oder Filters einschließt, wobei eine Substitutionsflüssigkeitsleitung (17, 19, 20) zu dem Blutkreislauf stromauf oder stromab des Dialysators oder Filters führt, in der eine Substituatpumpe (22) angeordnet ist, und

einer Einrichtung zur Bestimmung der Fistelrezirkulation,

**dadurch gekennzeichnet, dass**

die Einrichtung zur Bestimmung der Fistelrezirkulation aufweist:

    eine Steuereinheit (27) zum Abschalten und/oder Einschalten der Substituatpumpe,

eine Messeinheit (28) zum Messen des Drucks stromab des Dialysators oder Filters in der venösen Blutleitung des extrakorporalen Blutkreislauf und des Drucks stromauf des Dialysators oder Filters in der arteriellen Blutleitung des extrakorporalen Blutkreislauf, und eine Auswerteinheit (29), die derart ausgebildet ist, dass zur Bestimmung der Fistelrezirkulation das Verhältnis zwischen

dem Integral des in der arteriellen Blutleitung gemessenen arteriellen Drucksignals in einem vorgegebenen Zeitintervall nach dem Einschalten bzw. Ausschalten der Substituatpumpe, in dem ein sich wiederholender negativer bzw. positiver arterieller Druckpuls liegt, und
dem Integral des in der venösen Blutleitung gemessenen venösen Drucksignals in einem vorgegebenen Zeitintervall nach dem Einschalten bzw. Ausschalten der Substituatpumpe, in dem ein sich wiederholender negativer bzw. positiver venöser Druckpuls liegt,
berechnet wird.

4. Vorrichtung nach Anspruch 3, **dadurch gekennzeichnet, dass** die Messeinheit zum Messen des Drucks im Blutkreislauf (5A) stromauf und stromab des Dialysators (1) oder Filters einen arteriellen und venösen Drucksensor aufweist.

## Claims

1. A method for determining the fistula recirculation for a device for the extracorporeal treatment of blood with an extracorporeal blood circuit, which comprises a first chamber of a dialyser or filter divided by a membrane into the first chamber and a second chamber, and a fluid system which comprises the second chamber of the dialyser or filter, wherein substitution fluid is fed upstream or downstream of the dialyser or filter with a substitute pump, **characterized in that**
the substitute pump which had been shut-off is started up or the substitute pump which had been started up is shut-off, and the pressure is measured downstream of the dialyser or filter in the venous blood line of the extracorporeal blood circuit and the pressure is measured upstream of the dialyser or filter in the arterial blood line of the extracorporeal blood circuit and
for determining the fistula recirculation the ratio between
the integral of the pressure signal measured in the arterial blood line in a predetermined time interval after starting up and shutting off the substitute pump, respectively, in which a repeating negative and positive arterial pressure pulse, respectively, occurs, and
the integral of the pressure signal measured in the venous blood line in a predetermined time interval after starting up and shutting off the substitute pump, respectively, in which a repeating negative and positive venous pressure pulse, respectively, occurs,
is calculated.

2. The method according to claim 1, **characterized in that** the pressure is measured in the blood circuit downstream of the dialyser or filter with a pressure sensor whose output signal is filtered with a low-pass filter.

3. An apparatus for extracorporeal blood treatment comprising
an extracorporeal blood circuit (5A), which comprises a first chamber (3) of a dialyser (1) or filter divided by a membrane (2) into the first chamber and a second chamber (4), and a fluid system (5B) which comprises the second chamber of the dialyser or filter, wherein a substitution fluid line (17, 19, 20) leads to the extracorporeal blood circuit (5A) upstream or downstream of the dialyser or filter, in which line a substitute pump (22) is disposed, and
a device for determining the fistula recirculation, **characterized in that**
the device for determining the fistula recirculation comprises:

a control unit (27) for shutting off and/or starting up the substitute pump,
a measuring unit (28) for measuring the pressure downstream of the dialyser or filter in the venous blood line of the extracorporeal blood circuit and the pressure upstream of the dialyser or filter in the arterial blood line of the extracorporeal blood circuit, and an
evaluation unit (29), which is configured such that for determining the fistula recirculation the ratio between
the integral of the pressure signal measured in the arterial blood line in a predetermined time interval after starting up and shutting off the substitute pump, respectively, in which a repeating negative and positive arterial pressure pulse, respectively, occurs, and
the integral of the pressure signal measured in the venous blood line in a predetermined time interval after starting up and shutting off the substitute pump, respectively, in which a repeating negative and positive venous pressure pulse, respectively, occurs,
is calculated.

4. The apparatus according to claim 3, **characterized in that** the measuring unit for measuring the pressure in the blood circuit (5A) upstream or downstream of the dialyser (1) or filter comprises an arterial and venous pressure sensor.

## Revendications

**1.** Procédé de détermination de la recirculation dans une fistule pour un dispositif pour le traitement extracorporel du sang avec une circulation sanguine extracorporelle, qui inclut une première chambre d'un dialyseur ou d'un filtre subdivisé par une membrane dans la première chambre et une deuxième chambre, et un système de liquide qui inclut la deuxième chambre du dialyseur ou du filtre, dans lequel du liquide de substitution est acheminé en amont ou en aval du dialyseur ou du filtre avec une pompe de substitut, **caractérisé en ce que** la pompe de substitut désactivée est activée ou la pompe de substitut activée est désactivée, et la pression est mesurée en aval du dialyseur ou du filtre dans la conduite de sang veineux de la circulation sanguine extracorporelle et la pression est mesurée en amont du dialyseur ou du filtre dans la conduite de sang artériel de la circulation sanguine extracorporelle et **en ce que** pour la détermination de la recirculation dans une fistule, le rapport entre l'intégrale du signal de pression artérielle mesurée dans la conduite de sang artériel dans un intervalle temporel prédéterminé après l'activation ou l'arrêt de la pompe de substitut, dans lequel se situe une impulsion de pression artérielle négative ou positive se répétant, et l'intégrale du signal de pression veineuse mesurée dans la conduite de sang veineux dans un intervalle temporel prédéterminé après l'activation ou l'arrêt de la pompe de substitut, dans lequel se situe une impulsion de pression veineuse négative ou positive, est calculée.

**2.** Procédé selon la revendication 1, **caractérisée en ce que** la pression dans la circulation sanguine est mesurée avec un capteur de pression en aval du dialyseur ou du filtre, dont un signal de sortie est filtré avec un filtre passe-bas.

**3.** Dispositif pour le traitement extracorporel du sang avec une circulation sanguine extracorporelle (5A), qui inclut une première chambre (3) d'un dialyseur (1) ou d'un filtre subdivisé par une membrane (2) dans la première chambre et une deuxième chambre (4), et un système de liquide (5B) qui inclut la deuxième chambre du dialyseur ou du filtre, dans lequel une conduite de liquide de substitution (17, 19, 20) conduit à la circulation sanguine en amont ou en aval du dialyseur ou du filtre, dans lequel est disposée une pompe de substitut (22), et une installation pour la détermination de la recirculation dans une fistule,
**caractérisé en ce que**
l'installation pour la détermination de la recirculation dans une fistule présente :

une unité de commande (27) pour la désactivation et/ou l'activation de la pompe de substitut, une unité de mesure (28) pour la mesure de la pression en aval du dialyseur ou du filtre dans la conduite de sang veineux de la circulation sanguine extracorporelle et de la pression en amont du dialyseur ou du filtre dans la conduite de sang artériel de la circulation sanguine extracorporelle, et une unité d'évaluation (29) qui est conçue de telle sorte que pour la détermination de la recirculation dans une fistule, le rapport entre l'intégrale du signal de pression artérielle mesurée dans la conduite de sang artériel dans un intervalle temporel prédéterminé après l'activation ou l'arrêt de la pompe de substitut, dans lequel se situe une impulsion de pression artérielle négative ou positive se répétant, et l'intégrale du signal de pression veineuse mesurée dans la conduite de sang veineux dans un intervalle temporel prédéterminé après l'activation ou l'arrêt de la pompe de substitut, dans lequel se situe une impulsion de pression veineuse négative ou positive, est calculée.

**4.** Dispositif selon la revendication 3, **caractérisé en ce que** l'unité de mesure pour la mesure de la pression dans la circulation sanguine (5A) en amont ou en aval du dialyseur (1) ou du filtre présente un capteur de pression artérielle et veineuse.

**Fig. 1**

Fig. 2A

Fig. 2B

# Venenseitiger Druckverlauf während In Vitro HDF Dialysebehandlung

**Dialysegerät: 4008    Uf-Rate: 0 ml/h    Blutfluss: 250 ml/min    Pre/Post Sub-Rate: 70 ml/min**

**HDF Post-Dilution**

Zeit [min]    **Fig. 3A**

**HDF Pre-Dilution**

Zeit [min]    **Fig. 3B**

EP 2 238 996 B1

**Fig. 4**

## Fouriertransformierte:

| Norm. Koeffizienten der Signale der arteriellen Blutpumpe | 0,276 |
|---|---|
| Norm. Koeffizienten der Signale der Substituatpumpe | 0,403 |
| Auswertung | 0,403>>0,276=>post |

**Fig. 5A**

## Fouriertransformierte:

| Norm. Koeffizienten der Signale der arteriellen Blutpumpe | 0,265 |
|---|---|
| Norm. Koeffizienten der Signale der Substituatpumpe | 0,177 |
| Auswertung | 0,265≥0,177=>pre |

**Fig. 5B**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0189561 A
- EP 1348458 A
- DE 10115991 C1
- DE 19917197 C1